# EUROPEAN PATENT APPLICATION

(11) **EP 2 636 446 A1**
(43) Date of publication of application: **11.09.2013**
(21) Application number: 12158206.8
(22) Date of filing: 06.03.2012
(51) Int. Cl.: B01J 21/04, B01J 21/08, B01J 23/02, B01J 23/14, B01J 23/22, B01J 35/00, B01J 37/00, B01J 37/08, B01J 37/34, B01J 35/10, B01J 21/06, B05D 7/24

(54) **Plasma mediated method for producing catalysts**

(71) Applicant: VITO NV, 2400 Mol (BE); Katholieke Universiteit Leuven K.U. Leuven R&D, 3000 Leuven (BE)
(72) Inventor: Witvrouwen, Toon, B-2440 Geel (BE); Paulussen, Sabine, B-2100 Antwerpen-Deurne (BE); Sels, Bert, B-2490 Balen (BE)
(74) Representative: Bird Goën & Co

(57) **Abstract**

A method of producing an amorphous catalyst or catalyst precursor in the form of a powder having a surface area of from 0.1 m²/g to 50 m²/g which chemical composition comprises oxygen, carbon, hydrogen, optionally nitrogen, and at least a first and a second further elements, said method comprising:
a. Generating an aerosol comprising a mixture of derivatives, said mixture of derivatives comprising at least one derivative per further element, each derivative being a derivative of at least one of said further elements, and at least one of said derivatives being an organic derivative,
b. Diluting said aerosol by a gas, and
c. Passing said diluted aerosol in a plasma generated between two electrodes, at least one of which being provided with a dielectric, thereby forming an amorphous catalyst or catalyst precursor on one of said electrodes.

## Description

### Technical field of the invention

The present invention relates to a method for producing catalysts and catalysts precursors, as well as to the catalysts and catalysts precursors thereby obtained.

### Background of the invention

Conventional synthesis of heterogeneous catalysts often requires numerous steps and the use of solvents. This is for instance the case for the traditional synthesis of active tin-silicates for the isomerization of glucose into fructose. Moliner et al (PNAS, April 6, 2010, 107, 14, p. 6164-6168) describes for instance the synthesis of such a catalyst involving diluting a tetraethylammonium hydroxide solution, adding tetraethylorthosilicate and tin (IV) chloride pentahydrate, stirring the solution until complete hydrolysis, complete evaporation of ethanol and some water, adding a HF solution to form a gel, heating up the gel at 413 K for 40 days, filtrating the gel and washing the gel with water, drying the resulting solid and calcining the solid at 853 K for 6 hours.

Many other heterogeneous catalysts are synthesized by methods suffering from similar drawbacks as they are labor intensive and/or involve the use of solvents and/or are time consuming to produce.

There is therefore a need in the art for new, fast and not too labor intensive methods to produce catalysts, and in particular heterogeneous catalysts. There is also a need in the art for new catalyst and catalyst precursors that have good properties and are easy and fast to synthesize.

### Summary of the invention

It is an object of the present invention to provide a simple method to produce catalysts, and in particular heterogeneous catalysts. The present invention also provides new materials useful either as precursors to catalysts or as catalysts.

It is an advantage of embodiments of the present invention that the produced catalysts may have activities comparable to the activities of similar materials produced by traditional means.

The above objective is accomplished by a method and device according to the present invention.

In a first aspect, the present invention relates to an amorphous catalyst or to an amorphous catalyst precursor. The amorphous catalyst or catalyst precursor is in the form of a powder. In other words, in the first aspect, the present invention relates to a powder for use as a catalyst or as a precursor to a catalyst.

In the present invention, and unless provided otherwise, the term "precursor" refers to a powder, which after one or more further steps (typically a single calcination step), acquires catalytic properties or improved catalytic properties. This calcination step can be performed either before contacting the catalyst with the substrate or while contacting the catalyst with the substrate. This last case will be typical for a substrate reacting at a temperature at least equal to the temperature at which the calcination operates.

As used herein, and unless provided otherwise, the term "substrate" refers to the compound, the reaction of which the catalyst accelerates or enables.

In the present invention, the catalysts are typically used as heterogeneous catalysts.

In an embodiment of the first aspect, the present invention relates to an amorphous catalyst or catalyst precursor in the form of a powder having a surface area of from 0.1 m²/g to 50 m²/g which chemical composition comprises oxygen, carbon, hydrogen, optionally nitrogen, and at least a first and a second further element.

For instance, an embodiment of the first aspect may relates to an amorphous catalyst or catalyst precursor in the form of a powder having a surface area of from 0.1 m²/g to 50 m²/g which chemical composition comprises oxygen, carbon, hydrogen, a first further element, a second further element, and optionally nitrogen.

It is generally thought that inorganic heterogeneous catalyst comprising oxygen, and at least a first and a second further element (other than carbon, nitrogen or hydrogen) need to be crystalline or at least semi-crystalline to display a catalytic effect. It is also generally believed that a very high surface area is needed for a catalyst to be efficient.

The catalyst of the first aspect of the present invention surprisingly demonstrates high catalytic activity despite or because of them being simultaneously amorphous and having a relatively low surface area of from 0.1 m²/g to 50 m²/g. Without being bound by theory, it is believed that the amorphous state of the catalyst described herein is due to the presence of carbon and hydrogen in the structure of the catalyst. It is also believed that the carbon and hydrogen (and nitrogen when present) plays a positive role on the catalyst activity by improving its affinity for the substrate.

In an embodiment of the first aspect, the diameter of the powder particles may range from 0.05 to 8 µm, for instance from 0.1 to 4 µm or from 0.2 to 2 µm.

In an embodiment of the first aspect of the present invention, said at least a first and a second further element may be independently selected from the list consisting of metalloids, post-transition metals, transition metals, alkaline earth metal, non metals and alkali metals.

As used herein, and unless provided otherwise, the term *"metalloids"* encompasses the following elements: B, Si, Ge, As, Sb, and Te.

As used herein, and unless provided otherwise, the term *"post-transition metals"* encompasses the following elements: Al, Ga, In, Tl, Sn, Pb, and Bi.

As used herein, and unless provided otherwise, the term *"transition metals"* encompasses all lanthanides (i.e. elements having atomic numbers 57 to 71), all actinides (i.e. elements having atomic numbers 89 to 103), and all elements of group 3 to 12 of the periodic table (i.e. elements having atomic numbers 21 to 30, 39 to 48, 72 to 80 and 104 to 112). Preferred transition metals are elements of groups 4 and 5.

As used herein and unless provided otherwise, the term *"non-metal"* encompasses H, C, N, P, O, S, Se, F, Cl, Br, I and At. For the purpose of the present invention, the noble gases are not included in the definition of "non-metal". Also, in the context of the present invention, said at least a first and a second further elements cannot be any of H, C, N or O. Preferred non-metals are therefore P, S, Se, F, Cl, Br, I and At/ More preferred non-metals are P, S and Se. The most preferred non-metal is S.

As used herein and unless provided otherwise, the term *"alkali metal"* encompasses Li, Na, K, Rb, Cs, and Fr.

As used herein and unless provided otherwise, the term *"alkaline earth metal"* encompasses all elements of group 2.

The amorphous powders of the first aspect have a surface area of from 0.1 m²/g to 50 m²/g, preferably from 0.2 m²/g to 30 m²/g, more preferably from 0.3 to 25 m²/g and most preferably from 0.4 to 20 m²/g.

In an embodiment, said at least a first and a second further elements are a metalloid and a post-transition metal, a metalloid and a transition metal, a non-metal and a transition metal, an alkali metal and a transition metal, an alkaline earth metal and an alkali metal or a post-transition metal and an alkaline earth metal.

In embodiments, when the first and second further elements are a metalloid and a post-transition metal or metal-alkaline earth metal and post-transition metal, the powder may be more active or only active as a catalyst in its amorphous state (i.e. non calcined). In embodiments, when the first and second further elements are a metalloid and a post-transition metal or metal-alkaline earth metal and post-transition metal, the powder may be a catalyst.

In embodiments, when the first and second further elements are a metalloid and a transition metal, the powder may be more active or only active as a catalyst in its semi-crystalline form (i.e. calcined). In embodiments, when the first and second further elements are a metalloid and a transition metal, the amorphous powder may be a catalyst precursor.

In an embodiment of the present invention, said at least a first and a second further element may be Si and Sn, Si and V, Si and Zr, Si and Ti, S and Zr, K and Co, K and Ni, Li and Mg or Al and Mg.

In other embodiments of the present invention, said at least a first and a second further element are not Si and Ti.

In an embodiment of the present invention, said at least a first and a second further element may be Si and Sn, Si and V, Si and Zr, S and Zr, K and Co, K and Ni, Li and Mg or Al and Mg.

Embodiments of the present invention wherein said at least a first and a second further elements are Si and Sn or Al and Mg are advantageous as the present inventors could demonstrate that such powders have good catalytic activity (in the amorphous state) for the conversion of glucose into fructose.

Embodiments of the present invention wherein said at least a first and a second further elements are Si and V are advantageous since such powders are good candidate precursors (when in the amorphous state) to catalysts (in the calcined, semi-crystalline state) for the partial oxidation of methane.

Embodiments of the present invention wherein said at least a first and a second further elements are Zr and Si are advantageous since such powders are promising candidate precursors (when in the amorphous state) to catalysts (in the calcined, semi-crystalline state) for the isomerisation of n-butane into iso-butane.

It is an advantage of embodiments of the present invention that when the at least two further elements are Si and Sn, no calcinations is required to achieve good catalytic properties. SiSn catalysts of the prior art generally need calcination.

It is an advantage of embodiments of the present invention that when the at least two further elements are Al and Mg, no calcinations is required to achieve good catalytic properties.

In an embodiment, when said at least two further elements are Si and Sn, they may be in a ratio of from 5:1 to 250:1, preferably from 10:1 to 200:1, more preferably from 20:1 1 to 200:1, still more preferably from 100:1 1 to 200:1.

In an embodiment, when said at least two further elements are Si and Sn, C and Sn can be in a ratio of from 5:1 to 1750:1, preferably from 25:1 to 1500:1 and more preferably from 50:1 1 to 1000:1.

In an embodiment, when said at least two further elements are Si and Sn, N and Sn can be in a ratio of from 5:1 1 to 350:1, preferably from 5:1 1 to 200:1.

In an embodiment, in general, C and N may be in a ratio of from 5:1 1 to 1:1, preferably from 5:1 to 2:1. This for instance advantageous when said at least two further elements are Si and Sn.

In an embodiment, when said at least two further elements are Si and Sn, Sn may be predominantly tetracoordinated. When Sn is predominantly tetracoordinated, the catalyst leads to a better conversion and selectivity, and hence to a better yield. The predominantly tetracoordinated character of the Sn atoms can be determined by analysis of a UVDR spectra. Tetracoordinated Sn appears at 220±2 nm. In embodiments, the absorption of the Sn peak at 220±2 nm is from 1.7 to 1.9 times the absorption of the peak at 226±2 nm.

In an embodiment, the chemical composition of the amorphous catalyst or catalyst precursor may comprise from 10 to 70 at%, preferably from 20 to 50 at% carbon as measured by X-ray photoelectron spectroscopy or as measured by Energy-dispersive X-ray spectroscopy.

In an embodiment, the chemical composition of the amorphous catalyst or catalyst precursor may comprise from 0.04 to 2 at% tin as measured by X-ray photoelectron spectroscopy or as measured by Energy-dispersive X-ray spectroscopy.

X-ray photoelectron spectroscopy (XPS) is a surface characterization technique which does not provide information about the bulk of the catalyst. However, for a catalyst, it is the surface properties which are the most important. XPS is therefore a relevant technique. It is worth noting that both techniques (X-ray photoelectron spectroscopy and Energy-dispersive X-ray spectroscopy) do not measure hydrogen. The at% indicated here are therefore at% when hydrogen is not considered part of the composition (despite its actual presence). These ranges of at% have the effect to make the catalyst amorphous and to raise the affinity of the catalyst for an organic substrate, thereby providing good catalytic activity.

In a second aspect, the present invention relates to a method of producing an amorphous catalyst or catalyst precursor as defined in any embodiment of the first aspect. Said method comprises:
a) Generating an aerosol comprising a mixture of derivatives, said mixture of derivatives comprising at least one derivative per further element, each derivative being a derivative of at least one of said further elements, and at least one of said derivatives being an organic derivative,
b) Optionally diluting said aerosol by a gas, and
c) Passing said (optionally diluted) aerosol in a plasma generated between two electrodes, at least one of which being provided with a dielectric, thereby forming an amorphous catalyst or catalyst precursor on one of said electrodes.

This method is very advantageous as it permits to generate catalysts or catalyst precursor in a matter of minutes via a simple procedure.

In an embodiment, step (a) of the method may comprise generating an aerosol comprising a mixture of organic derivatives, said mixture of organic derivatives comprising at least one organic derivative per further element, each organic derivative being an organic derivative of at least one of said further elements,

The step of generating an aerosol can be performed by using one or more atomizers. This can be done in several ways as explained below.

In an embodiment, said aerosol may be generated by passing one or more liquids, each comprising one or more of said derivatives, in one or more atomizer. For instance, each derivative can be used pure (e.g. if liquid) or put in solution (e.g. if solid or to reduce the viscosity in case of viscous liquids) in a different flask, before to lead each liquid to a different atomizer. Another possibility is to put all derivatives in the same flask (with an optional solvent) and to lead this mixture to an atomizer. Yet another possibility is to put each derivative (as a pure liquid or in solution) in different flasks and to lead each flask to a separate atomizer.

In an embodiment, said aerosol may be generated by using nitrogen as an aerosol gas, wherein said gas used for the optional dilution of the aerosol is nitrogen and wherein said plasma gas is nitrogen. However, any other gas and mixture of gases can be used as well. For instance, the following gases (and optionally mixtures thereof) can be used: noble gases, H₂, O₂, CO₂, CO, NH₃, N₂ and CH₄. Preferably, the gas is selected from N₂, He, Ar, O₂, CO₂, H₂ and optionally mixtures thereof.

The use of nitrogen as a gas (whether as an aerosol gas, a dilution gas and/or a plasma gas) leads to incorporation of nitrogen in the structure of the catalyst.

The nature of the organic part of the organic derivative is not crucial as long as it is organic, i.e. it comprises hydrogen and carbon atoms (and optionally other atoms such as e.g. oxygen. Preferably, each derivative is an organic derivative.

In an embodiment, the said organic derivatives of said first and second elements may be respectively tetra ethyl orthosilicate and titanium tetra isopropoxide, tetra ethyl orthosilicate and tetra-n-butyltin, tetra ethyl orthosilicate and vanadium (V) oxytripropoxide, tetra ethyl orthosilicate and zirconium n-propoxide, or aluminium lactate and magnesium acetate tetrahydrate.

These embodiments lead to catalysts and catalysts precursors described in the first aspect of the present invention wherein said at least a first and a second further element may be Si and Ti, Si and Sn, Si and V, Si and Zr, or Al and Mg.

In an embodiment, the said organic derivatives of said first and second elements may be respectively tetra ethyl orthosilicate and tetra-n-butyltin, tetra ethyl orthosilicate and vanadium (V) oxytripropoxide, tetra ethyl orthosilicate and zirconium n-propoxide, or aluminium lactate and magnesium acetate tetrahydrate.

In an embodiment, the said organic derivatives of said first and second elements are not tetra ethyl orthosilicate and titanium tetra isopropoxide.

The optional step of diluting said aerosol by a gas can for instance be performed by contacting the aerosol with a controlled mass flow of gas.

The step of passing said (optionally diluted) aerosol in a plasma generated between two electrodes can be simply performed by directing said (optionally diluted) aerosol toward the space present between two electrodes of plasma reactor.

In an embodiment, the plasma reactor may be suitable for use in a dielectric barrier discharge methodology. This methodology involves an electrical discharge between two electrodes separated by an insulating dielectric barrier. The feature typically found in a plasma reactor suitable for use in a dielectric barrier discharge methodology are therefore a power source, two electrodes and at least one dielectric barrier between said electrodes.

The electrodes are preferably flat electrodes so that the powder produced can be more easily collected. The electrodes can be controlled in temperature (e.g. water cooled).

In embodiments, said plasma may be generated in a chamber at atmospheric pressure and at a temperature lower than 200°C, preferably lower than 60°C. This is advantageous since little energy in necessary for the synthesis and means for heating the reaction chamber are not necessary.

The frequency of the electrical field used to generate the plasma does not have a large influence on the yield of catalyst obtained. A wide range of frequency can therefore be used. For instance, it can be from 0.1 to 200 kHz, preferably from 1 to 100 kHz, more preferably from 3 to 60 kHz and most preferably from 4 to 23 kHz.

In embodiments, the frequency of the electrical field may be from 3 to 60 kHz, preferably 4 to 23 kHz. The inventors have determined that in the case of the production of Sn silicate catalysts (see example 1), a lower frequency was typically associated with a higher selectivity. For the other catalysts exemplified below, similar observations were made. It might therefore be advantageous to work at lower frequency in order to obtain catalysts leading to a catalysis product easier to purify. For instance, frequencies of 11 kHz or lower will typically lead to a higher selectivity than a frequency above 11 kHz. A frequency of 8 kHz or lower may be advantageous to favour high selectivity.

A wide range of power for the electrical field can be used. In embodiments, the power of the electrical field may be from 0.02 W/cm² to 0.65W/cm².

In the case of the production of Sn silicate catalysts (see example 1), the power of the electrical field appears to be correlated with catalysts having higher conversion rate, selectivity and therefore yields. Good catalysts have however been obtained at relatively low power. For other catalysts exemplified below, similar observations were made.

In embodiment, a power of 0.09 W/cm² W or higher, preferably 0.15 W/cm² W or higher, more preferably 0.22 W/cm² or higher or even 0.48 W/cm² or higher may be used. For instance, the power can be selected between 0.09 and 0.48 W/cm².

The gas flow of the dilution gas can take a large window of values. For instance it can be from 0 to 100 standard liters per minute (slm) and preferably from 10 to 40 slm. The dilution gas is optional.

In the case of the production of Sn silicate catalysts (see example 1), the extent of the gas flow of the dilution gas appears to be correlated with catalysts having higher conversion rate, selectivity and therefore yields. Good catalysts have however been obtained at relatively low dilution gas flow. For the other catalysts exemplified below, similar observations were made.

In embodiments, a gas flow of 15 slm or higher, preferably 18 slm or higher, more preferably 22 slm or higher, still more preferably 27 slm or higher can be used. For instance, a dilution gas flow of from 18 slm to 27 slm can be used.

In embodiments, the method of the second aspect may further comprise, after step (c), a step (d) of removing said amorphous catalyst or catalyst precursor from said electrode. Preferably, the electrode on which the powder is collected is provided with said dielectric. In this embodiment, the powder is removed from the surface of said dielectric.

In embodiments, the method of the second aspect may further comprise a step (e) after step (d) of calcining said amorphous catalyst precursor. For some powders obtained in step (c) (catalyst precursors), a calcining step is necessary to obtain or to increase catalytic properties.

In a third aspect, the present invention relates to an amorphous catalyst or catalyst precursor obtainable by any method of the second aspect of the present invention.

In a fourth aspect, the present invention relates to a semi-crystalline catalyst obtainable by calcination of the amorphous catalyst precursor of any embodiments of the first or third aspect of the present invention.

In a preferred embodiment of the fourth aspect, the first and second further element may be a metalloid and a transition metal.

In a fifth aspect, the present invention relates to the use of an amorphous catalyst according to any embodiment of the first or third aspect , wherein said at least a first and a second further element are Si and Sn or Al and Mg, for catalysing the isomerisation of glucose into fructose.

In a sixth aspect, the present invention relates to the use of the catalyst according to any embodiment of the fourth aspect, wherein said at least a first and a second further element are Si and V, for catalyzing the partial oxidation of methane.

In a seventh aspect, the present invention relates to the use of the catalyst according to any embodiment of the fourth aspect, wherein said at least a first and a second further element are Si and Zr, for catalyzing the isomerisation of n-butane into iso-butane.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a scheme of an apparatus used for performing a method according to an embodiment of the second aspect of the present invention.
Fig. 2 shows the conversion of glucose in function of time for catalysts according to embodiments of the first aspect of the present invention.
Fig. 3 shows UVDR spectra of catalysts according to embodiments of the first aspect of the present invention.
Fig. 4 shows an XPS spectrum demonstrating the presence of carbon in a catalyst according to embodiments of the first aspect of the present invention.
Fig. 5 is a XRD spectrum demonstrating the amorphous nature of a catalyst according to embodiments of the first aspect of the present invention.
Fig. 6 is a XRD spectrum demonstrating the semi-crystalline nature of a catalyst according to embodiments of the fourth aspect of the present invention.
Fig. 7 is another XRD spectrum demonstrating the semi-crystalline nature of a catalyst according to embodiments of the fourth aspect of the present invention.
Fig. 8 shows the conversion of glucose in function of time for catalysts according to embodiments of the first aspect of the present invention.
Fig. 9 is a XRD spectrum demonstrating the amorphous nature of a catalyst according to embodiments of the first aspect of the present invention.
Fig. 10 is a Fourier Transform Infrared Spectrum of a catalyst according to embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

### Examples

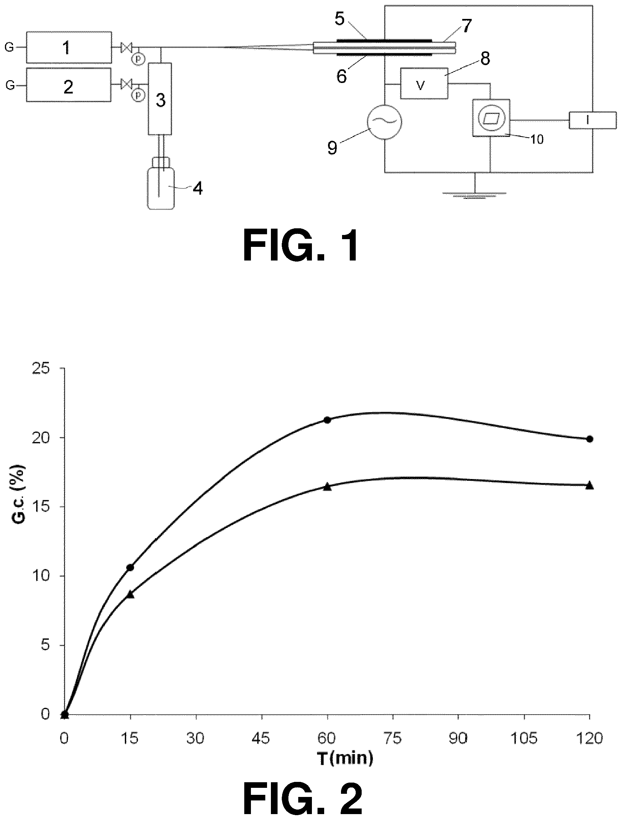

All synthesis referred to in the examples below have been performed in a plasma reactor as shown in Fig. 1.

In Fig. 1, a plasma reactor is shown. It uses the dielectric barrier discharge (DBD) methodology. The plasma reactor comprises two flat water-cooled aluminum electrodes (5, 6) of 460 cm² each, both covered by 3.3 mm thick borofloat glass plates (7) (Glastec, Belgium) serving as dielectrics. A gap of 3 mm between the two glass plates (7) is held constant during all experiments. While one electrode is earthed (5), the other (6) is connected to a high voltage transformer (8) (TR12/1TU-V, AFS, Germany) which is coupled to a 1:1000 high-voltage generator (9) (G10S-V, AFS, Germany). An optional oscilloscope (10) is attached to the reactor to be able to analyze the voltage and current signal, and to calculate the input power the generator (9) is sending to the electrodes. Several mass flow controllers (1, 2) are utilized to bring a gas G (e.g. nitrogen) to the reactor and atomizer(s) (3), used for the creation of aerosols from the precursor liquid(s) (4). Only two mass flow controllers (1, 2) (MKS Instruments, USA) are depicted for the sake of clarity but actually more of them can be used. Typically, one mass flow controller (1) is used for the optional dilution step and a separate flow controller (2) is used for each precursor-containing flask. The atomizers input can also be selected or controlled to regulate the precursor input. The gas (G) used was nitrogen for all purposes (as a plasma gas, a dilution gas and an aerosol gas) and all experiments.

In operation, an aerosol of the used precursors (4) was generated in the atomizer(s) (3). The aerosol, diluted by the plasma gas (G), was guided between the two electrodes (5, 6), on which the materials were deposited after reaction in the non-equilibrium plasma. The powder was scraped and collected from the glass plates (7) for analysis.

### Example 1: Synthesis of Sn-Si catalysts

Tetraethylorthosilicate (TEOS, ≥ 99%, Merck) and tetra-n-butyltin (95%, Alfa Aesar) were used as precursor reagents for the synthesis of the tin-silicate based catalysts.

A mixture of aerosols of the precursor fluids (4), resulting into tin-silicate catalysts with a surface Si/Sn ratio around 17, was brought in the plasma zone after dilution with nitrogen gas (G). Catalyst powder was deposited on the flat plates (7) because of the polymerization reactions that occurred in the chemically reactive non-equilibrium plasma. The samples were scraped from the glass plates (7) and collected for characterization and analysis of the catalytic properties.

First, a design of experiment (DoE) approach was used to select the synthesis parameters for the DoE-catalysts to be able to extract as much information as possible out of the effect of the selected parameters (plasma power, plasma frequency and the amount of dilution gas) on the catalytic properties of the synthesized powders, while minimizing the amount of experiments. The Design Expert 8 (Stat-Ease, Inc., USA) software was used as a tool to create and analyze the design and model. A globular, three-level Box-Bhenken design does not include too extreme factors, which might be difficult to obtain in the plasma reactor, and was therefore chosen for the analysis. The selected design points are given in the table below and are named DoEx, with x being the run order of the catalyst synthesis.

| *Box-Behnken design points.* | | | |
|---|---|---|---|
| **Name** | **Power** | **Frequency** | **Dilution Gas** |
| | **(W/cm²)** | **(kHz)** | **(slm N₂)** |
| DoE₁ | 0.09 | 11 | 18 |
| DoE₂ | 0.15 | 8 | 18 |
| DoE₃ | 0.15 | 11 | 22.5 |
| DoE₄ | 0.09 | 14 | 22.5 |
| DoE₅ | 0.15 | 14 | 18 |
| DoE₆ | 0.15 | 11 | 22.5 |
| DoE₇ | 0.22 | 11 | 18 |
| DoE₈ | 0.22 | 14 | 22.5 |
| DoE₉ | 0.15 | 14 | 27 |
| DoE₁₀ | 0.15 | 8 | 27 |
| DoE₁₁ | 0.09 | 11 | 27 |
| DoE₁₂ | 0.15 | 11 | 22.5 |
| DoE₁₃ | 0.22 | 11 | 27 |
| DoE₁₄ | 0.22 | 8 | 22.5 |
| DoE₁₅ | 0.15 | 11 | 22.5 |
| DoE₁₆ | 0.09 | 8 | 22.5 |

This design gave the ability to analyze the response factors (activity, selectivity, and yield), within the selected parameter field (plasma power: 0.09 - 0.22W/cm², frequency: 8 - 14kHz, N₂ gas flow: 18 - 27slm).

Several more catalysts were synthesized to verify the resulting model outside the previously defined parameter field, but also to investigate the effect of a changing Si/Sn input ratio. The table below shows the synthesis conditions for these additionally produced catalysts, named SiSn^{x}. The Si input flow is always 1.7 slm for each of the two TEOS atomizers. Sn input flows of 0.29 slm, 0.32 slm, 0.36 slm, 0.40 slm and 0.45 slm are used on respectively SiSn¹, SiSn², SiSn³, SiSn⁴ and SiSn⁵ respectively.

| *Synthesized SiSn^{x} catalysts (21 slm N₂).* | | | |
|---|---|---|---|
| **Name** | **Power** | **Frequency** | **Remarks** |
| | **(W/cm²)** | **(kHz)** | |
| SiSn¹ | 0.48 | 23 | 19% less Sn-precursor input flow |
| SiSn² | 0.48 | 23 | 11% less Sn-precursor input flow |
| SiSn³ | 0.48 | 23 | Same Si & Sn precursor input flow as DoE-catalysts |
| SiSn⁴ | 0.48 | 23 | 11% more Sn-precursor input flow |
| SiSn⁵ | 0.48 | 23 | 25% more Sn-precursor input flow |

In embodiments, the Si to Sn precursor input flow ratio can be from 0.1 to 200, preferably from 10 to 200, more preferably 50 to 200 and most preferably from 100 to 150.

### Characterization

A field emission gun scanning electron microscope (JSM-6340F, JEOL) with a conventional detector for secondary electrons (LEI) was used to obtain images and evaluate the microscopic structure and estimate the diameter of the catalyst particles.

The diameter was thereby estimated to be ranging from 0.2 to 2 µm.

The Si/Sn ratio was determined by XPS analysis using a theta probe - high performance XPS Spectrometer (Thermo Electron Corporation, United States). The operation was carried out with a pass energy of 100 eV and monochromatic Al Kα X-radiation with photon energy of 1486.6 eV.

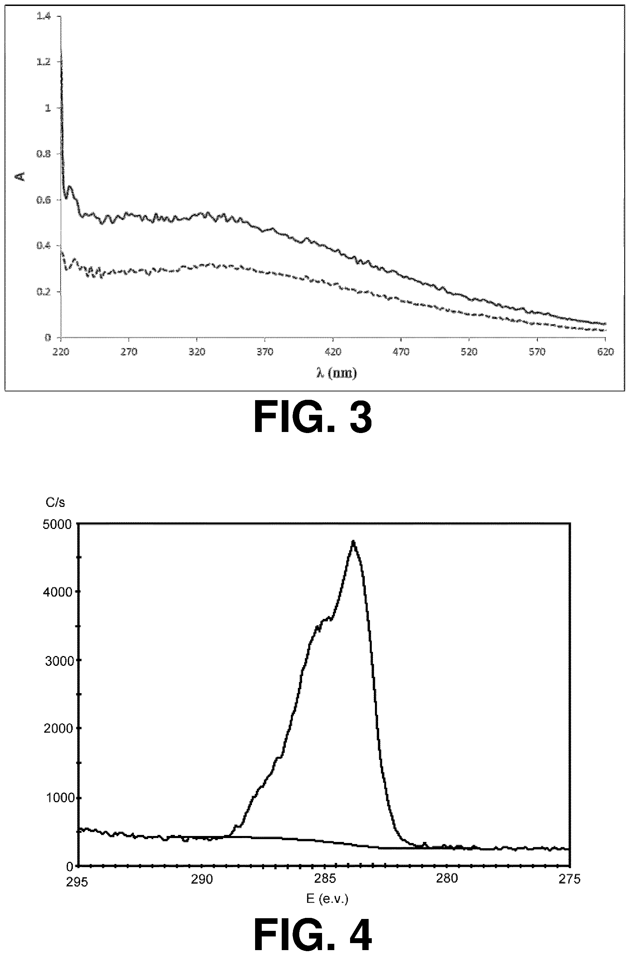

Fig. 4 is an example of XPS spectrum indicating the presence of carbon in a SiSn catalyst according to embodiements of the present invention.

The XPS analysis for samples SiSn¹ to SiSn⁵ are summarized below:

| SiSn¹ | | | | | | |
|---|---|---|---|---|---|---|
| Si | 11,33 | 12,75 | 12,77 | 15,18 | 15,07 | |
| Sn | 0,57 | 0,76 | 0,61 | 0,85 | 0,82 | |
| C | 46,95 | 43,33 | 43,74 | 38,3 | 39,98 | |
| N | 14,12 | 13,39 | 13,4 | 12,6 | 11,97 | averages |
| **Si/Sn** | 19,88 | 16,78 | 20,93 | 17,86 | 18,38 | **19,26** |
| **C/Sn** | 82,37 | 57,01 | 71,70 | 45,06 | 48,76 | 60,98 |
| **N/Sn** | 24,77 | 17,62 | 21,97 | 14,82 | 14,60 | 18,76 |
| **C/N** | 3,33 | 3,24 | 3,26 | 3,04 | 3,34 | 3,24 |

| SiSn² | | | | |
|---|---|---|---|---|
| Si | 10,12 | 8,28 | 10,38 | |
| Sn | 0,09 | 0,05 | 0,07 | |
| C | 45,29 | 51,38 | 43,81 | |
| N | 19,02 | 16,21 | 20,59 | averages |
| **Si/Sn** | 112,44 | 165,6 | 148,29 | **130,37** |
| **C/Sn** | 503,22 | 1027,6 | 625,86 | 718,89 |
| **N/Sn** | 211,33 | 324,2 | 294,14 | 276,56 |
| **C/N** | 2,38 | 3,17 | 2,13 | 2,56 |

| SiSn³ | | | | |
|---|---|---|---|---|
| Si | 11,56 | 11,43 | 8,12 | |
| Sn | 0,1 | 0,09 | 0,04 | |
| C | 42,43 | 43,87 | 53,58 | |
| N | 18,13 | 17,81 | 13,88 | averages |
| **Si/Sn** | 115,6 | 127 | 203 | **148,53** |
| **C/Sn** | 424,3 | 487,44 | 1339,5 | 750,41 |
| **N/Sn** | 181,3 | 197,89 | 347 | 242,06 |
| **C/N** | 2,34 | 2,46 | 3,86 | 2,89 |

| SiSn⁴ | | | | | | |
|---|---|---|---|---|---|---|
| Si | 9,46 | 9,35 | 12,14 | 11,87 | 11,5 | |
| Sn | 0,64 | 0,57 | 0,73 | 0,77 | 0,73 | |
| C | 52,45 | 54,41 | 45,97 | 45,87 | 47,33 | |
| N | 11,48 | 10,73 | 12,95 | 13,24 | 12,54 | averages |
| **Si/Sn** | 14,78 | 16,40 | 16,63 | 15,42 | 15,75 | **16,05** |
| **C/Sn** | 81,95 | 95,46 | 62,97 | 59,57 | 64,84 | 72,96 |
| **N/Sn** | 17,94 | 18,82 | 17,74 | 17,19 | 17,18 | 17,77 |
| **C/N** | 4,57 | 5,07 | 3,55 | 3,46 | 3,77 | 4,086 |

| SiSn⁵ | | | | | | |
|---|---|---|---|---|---|---|
| Si | 18,09 | 17,47 | 16,74 | 16,21 | 16,03 | |
| Sn | 1,23 | 1,42 | 1,19 | 1,14 | 1,33 | |
| C | 35,9 | 36,85 | 37,08 | 39,1 | 38,1 | |
| N | 7,65 | 8,27 | 8,31 | 8,17 | 9,27 | averages |
| **Si/Sn** | 14,71 | 12,30 | 14,07 | 14,22 | 12,05 | **13,16** |
| **C/Sn** | 29,19 | 25,95 | 31,16 | 34,30 | 28,65 | 29,85 |
| **N/Sn** | 6,22 | 5,82 | 6,98 | 7,167 | 6,97 | 6,63 |
| **C/N** | 4,69 | 4,456 | 4,46 | 4,79 | 4,11 | 4,50 |

In the tables above, one table is for one catalyst powder. The different columns within one table are measurement made on the same catalysts but at different points. These compositions represent the relative atomic proportions of the elements measured. It does not represent the actual composition of the material since e.g. H is not measured by this method. The ratios are however of particular relevance.

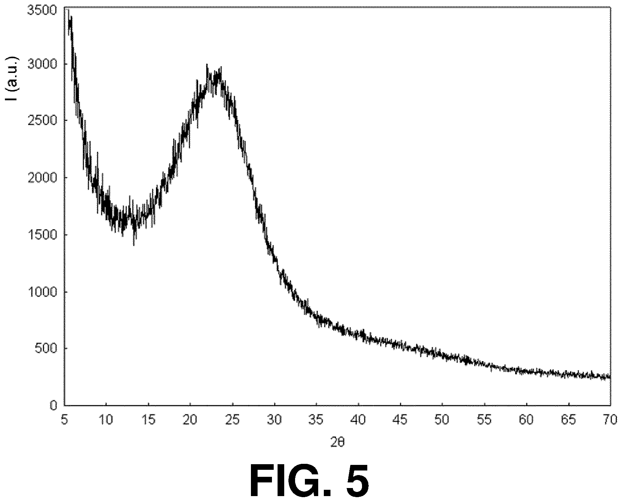

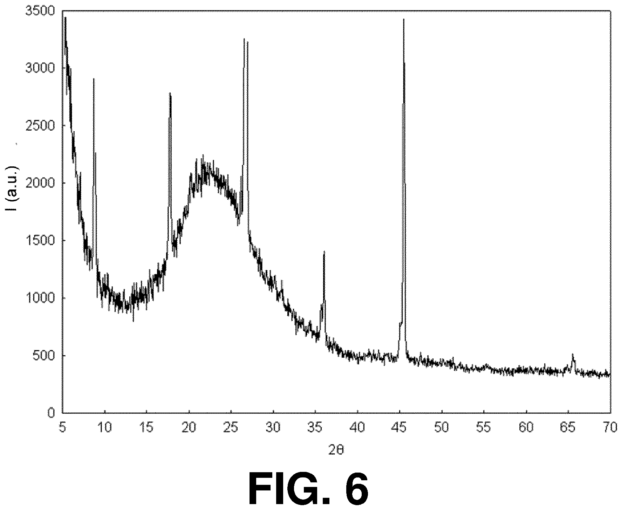

Fig. 5 is a XRD spectrum of the catalyst showing its amorphous nature (no peaks are visible that would indicate a crystalline or semi-crystalline state). This is to compare with the situation of Fig. 6 of example 3.

UV-VIS-DR spectra were recorded on a UV-VIS spectrophotometer (Thermo Electron Evolution 500), equipped with an integrating sphere (RSA-UC-40 diffuse reflectance cell). The average of three scanning cycles was taken with a scanning speed of 120 nm/min and a band width of 2 nm. Samples were diluted to 2 wt% with dry KBr and pure KBr was used as background.

The surface area of powder samples was measured via Multipoint BET measurements (Quantachrome Instruments, Nova). All samples went through the same outgas procedure of 20 hours at 110 °C to remove water prior to measurement. The measurements made are summarized in the table below:

| Sample | BET surface area (m²/g) |
|---|---|
| DoE7 | 8.37 |
| DoE10 | 16.87 |
| DoE12 | 5.22 |
| DoE15 | 12.07 |

The catalytic properties of the catalysts for the glucose isomerization reaction were tested in glass flasks filled with 10 wt% glucose and 20 mg of catalyst in water. These solutions were brought to 383 K. Samples were taken for HPLC analysis several times to be able to calculate the corresponding activity, selectivity, and yield.

Figure 2 shows the glucose conversion in function of time for DoE₁₁ (triangles) and DoE₁₂ (circles).

The table below shows the corresponding conversions, selectivities and yields for the DoEx of the SiSnx catalysts. The conversion, selectivity, and yield for all DoE catalysts was calculated after one hour; for most DoE-catalysts, the maximum conversion was reached at that time.

| *Response factors after 60 minutes of reaction (21 slm N₂). *Reaction time is 120 min instead of 60.* | | | |
|---|---|---|---|
| **Name** | **Glucose isomerization** | | |
| | **Conversion (%)** | **Selectivity (%)** | **Fructose Yield (%)** |
| DoE₁ | 10.5 | 58.4 | 6.1 |
| DoE₂ | 13.5 | 82.4 | 11.1 |
| DoE₃ | 16.4 | 76.7 | 12.6 |
| DoE₄ | 17.4 | 67.1 | 11.7 |
| DoE₅ | 14.5 | 74.5 | 10.8 |
| DoE₆ | 16.0 | 75.0 | 12.0 |
| DoE₇ | 16.4 | 83.0 | 13.6 |
| DoE₈ | 19.2 | 68.9 | 13.2 |
| DoE₉ | 17.0 | 69.5 | 11.8 |
| DoE₁₀ | 12.8 | 92.2 | 11.8 |
| DoE₁₁ | 16.5 | 73.0 | 12.0 |
| DoE₁₂ | 17.8 | 75.0 | 13.4 |
| DoE₁₃ | 17.4 | 69.0 | 12.0 |
| DoE₁₄ | 15.3 | 86.8 | 13.3 |
| DoE₁₅ | 17.7 | 67.2 | 11.9 |
| DoE₁₆ | 14.3 | 78.3 | 11.2 |
| SiSn₁* | 21.2 | 69.7 | 14.7 |
| SiSn₂* | 23.4 | 71.5 | 16.7 |
| SiSn₃* | 22.5 | 70.1 | 15.8 |
| SiSn₄* | 20.4 | 65.4 | 13.3 |
| SiSn₅* | 13.9 | 60.4 | 8.4 |

The spectra, obtained by the UV-VIS diffuse reflectance technique, for some of the DoE catalysts (Fig. 3, DoE8 is the solid line and DoE1 is the dashed line) show a clear peak at 220 ± 2 nm (O²⁻ → Sn⁴⁺), which demonstrates the presence of Sn⁴⁺ in the tetrahedral coordination and thus more active. However, between the worst and best performing catalysts, a big difference can be seen in the intensity of the absorbance around 220 nm; the more active catalysts have a larger concentration of tetrahedral coordinated tin species than the ones with a lower absorption bands around 220 nm. The absorbance at higher wavelengths can be attributed to hexacoordinated Sn-O-Sn species: around 240 nm for isolated Sn-O-Sn species and above 280 nm for polymeric SnO₂ species.

The calculated catalytic properties of the DoE catalysts were analyzed by Design- Expert 8 and the resulting model for activity, selectivity and yield, is given in the table below.

| *Model for conversion, selectivity, and yield for DoE tin-silicate catalysts with the range of 0.09-0.22 W*/*cm² plasma power, a frequency of 8-14 kHz, and a gas flow between 18 and 27 slm (A* = *plasma power (W*/*cm²), B* = *frequency (kHz), C* = *gas flow (slm)).* | | | | | | | | | | |
|---|---|---|---|---|---|---|---|---|---|---|
| | **Intercept** | **A** | **B** | **C** | **AB** | **AC** | **BC** | **A²** | **B²** | **C²** |
| Conversion (%) | -60,19 | 114.62 | 0,51 | 5,20 | 0 | -4.27 | 0 | 0 | 0 | 0,0956 |
| Selectivity (%) | -10,45 | 861.26 | -9,28 | 6,88 | 0 | -24.44 | -0,27 | -828.40 | 0,59 | 0 |
| Yield (%) | -48.07 | 165.58 | 0 | 3.95 | 0 | -6.41 | 0 | 0 | 0 | -0.062 |

According to the model, valid within the range of synthesis conditions, a high plasma power and frequency leads to the most active catalysts (high conversion). However, when we look at selectivity, a low frequency is advantageous as well as a medium to high plasma power. Combining the activity and selectivity in yield, then we can see that the plasma frequency has nearly no influence on the overall yield of the catalyst.

The conclusions of the model were verified by synthesis of catalysts at higher plasma power and higher frequency (SiSn¹ till SiSn⁵). A total gas flow of 21 slm N₂ was chosen for these experiments. Not only plasma power and frequency were further investigated, but also a changing Si/Sn input ratio. The gas flow on the atomizer of the tin precursor was varied until the optimal amount of tin precursor was incorporated into the silicate structure, which, in this case, corresponds with an actual surface Si/Sn ratio of 130, resulting in a fructose yield of 16.7%. The yield was still increasing after one hour of reaction, so an extra hour was added to the reaction time, indicating that the stability of the catalysts was also enhanced compared to the previously tested DoE catalysts.

### Example 2: Synthesis of a Ti-Si catalyst

Tetraethylorthosilicate (TEOS, ≥ 99%, Merck) and titanium tetraisopropoxide were used as precursor reagents for the synthesis of the titanium silicate based catalysts. The synthesis proceeded analogously to example 1 with the following parameters: a frequency of 9 kHz, a power of 50 W (i.e. 0.11 W/cm²), a precursor flow rate of 22.5 slm (TEOS and Titanium tetraisopropoxide were mixed in a single bottle).

In embodiments, this synthesis preferably performed at a frequency of from 4 to 40 kHz. In embodiments, this synthesis preferably performed at a power of from 40 to 175 W (ie. from 0.09 to 0.38 W/cm²). In embodiments, this synthesis is preferably performed with an aerosol flow rate of from 10 to 30 slm.

### Characterization:

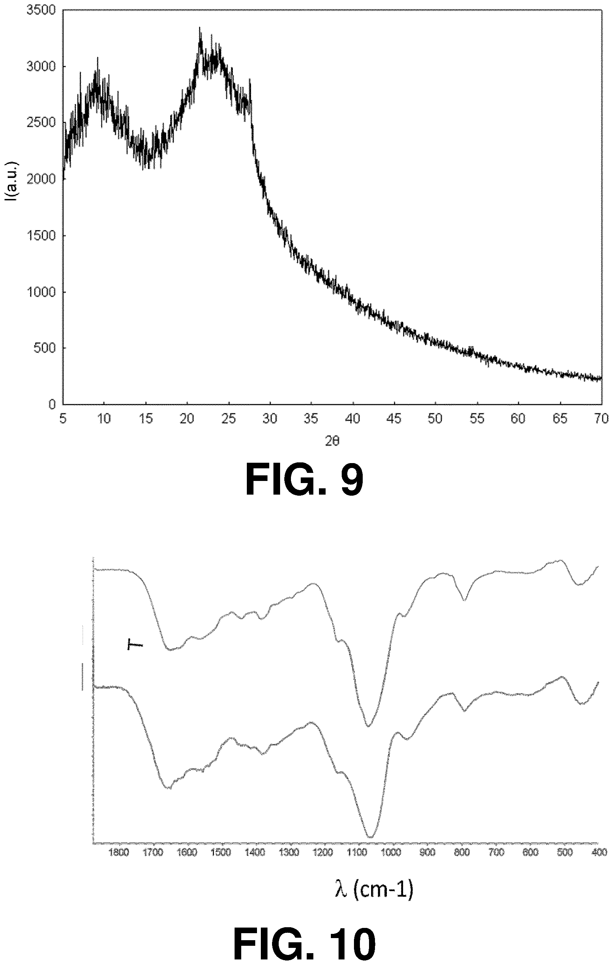

A comparison between powders, synthesized with (Si/Ti=120), and without titanium was made by Fourier Transform Infrared Spectroscopy (FTIR) (see Fig. 10). A shift of the Si-O-S stretch from 1075cm⁻¹ to 1070cm⁻¹ could be observed after incorporation of the titanium.

The catalytic properties of the catalysts for the epoxidation of cyclooctene were tested and 56% of the cyclooctene were converted.

### Example 3: Synthesis of a V-Si catalyst

Three precursor bottles, i.e. two containing tetraethylorthosilicate (TEOS, ≥ 99%, Merck) and one containing vanadium (V) oxytripropoxide (Aldrich, 98%) were used as precursor reagents for the synthesis of the vanadia silicate based catalysts. The synthesis proceeded analogously as described for example 1 but with a frequency of 23 kHz, 25 slm of total gas flow and a power of 150W (0.33 W/cm²). The resulted catalyst precursors were used after calcinations at 500°C.

The presence of silicium, carbon, vanadium, nitrogen and oxygen in the structure of the powder (before calcinations) was confirmed by EDX. After calcinations, the amount of carbon and nitrogen was greatly reduced.

Fig. 6 is a XRD spectrum of the catalyst showing its semi-crystalline nature after calcination (peaks are visible in addition to the global featureless shape. This indicates a semi-crystalline state). This is to compare with the situation of Fig. 5 of example 1.

The surface area was measured as indicated for example 1 and amounted to 12.72 m²/g.

### Example 4: Synthesis of a Zr-Si catalyst

One precursor bottle comprising a mixture of tetraethylorthosilicate (TEOS, ≥ 99%, Merck, 21.37g) and zirconium n-propoxide (70% in propanol, ABCR; 12g) was used as a precursor mixture for the synthesis of the zirconia silicate based catalysts. The synthesis proceeded as described for example 1 and the resulted catalyst precursors were used after calcinations at 650°C.

The obtained calcined powder is a candidate catalyst for the isomerisation of n-butane into iso-butane. Without being bound by theory, this is likely due to the presence of the tetragonal zirconia crystals.

The presence of silicium, carbon, Zirconium, nitrogen and oxygen in the structure of the powder (before calcinations) was confirmed by XPS. After calcinations, the amount of carbon and nitrogen was greatly reduced.

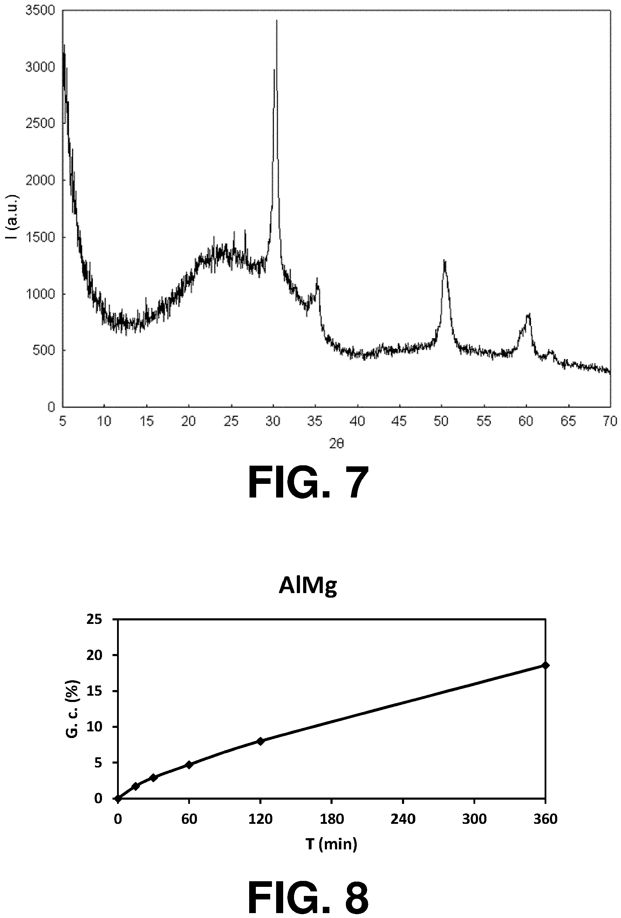

Fig. 7 is a XRD spectrum of the catalyst showing its semi-crystalline nature after calcination (peaks are visible in addition to the global featureless shape. This indicates a semi-crystalline state).

The surface area was measured as indicated for example 1 and amounted to 18.24 m²/g.

### Example 5: Synthesis of an Al-Mg catalyst

One precursor bottle comprising a mixture of magnesium acetate tetrahydrate (pure, crystalline, Acros, 1.86 g) and aluminium lactate (0.57g) was brought into a solution with demineralized water (18 ml) and methanol (Merck, ≥99.9%, 30 ml) used as a precursor mixture for the synthesis of the alumina magnesia based catalysts. The synthesis proceeded as described for example 1 and the resulted catalyst precursors were used without calcination.

The catalytic properties of the catalysts for the glucose isomerization reaction were tested as described for example 1. Fig. 8 shows the glucose conversion (%) over time.

The presence of, carbon, Mg, Al, nitrogen and oxygen in the structure of the powder (before calcinations) was confirmed by EDX.

Fig. 9 is a XRD spectrum of the catalyst showing its amorphous nature (no peaks are visible that would indicate a crystalline or semi-crystalline state).

The surface area was measured as indicated for example 1 and amounted to 19.04 m²/g.

### Example 6: Synthesis of an S-Zr (sulfated zirconia) catalyst

### First procedure:

One precursor bottle of Zr-n-propoxide and one bottle with a solution of sulfuric acid is used as precursor reagents for the synthesis of the S-Zr based catalysts.

A procedure analogous to the procedure of example 1 is followed to obtain a sulfated zirconia catalyst. The powder obtained is a candidate catalyst for the isomerization of n-butane into isobutene.

### Second procedure:

One precursor bottle of Zr-n-propoxide and one bottle with a solution of potassium sulphate is used as precursor reagents for the synthesis of the S-Zr based catalysts.

A procedure analogous to the procedure of example 1 is followed to obtain a sulfated zirconia catalyst. The powder obtained is a candidate catalyst for the isomerization of n-butane into isobutene.

### Third procedure:

One precursor bottle of Zr-n-propoxide and one bottle with ethyl sulfate is used as precursor reagents for the synthesis of the S-Zr based catalysts.

A procedure analogous to the procedure of example 1 is followed to obtain a sulfated zirconia catalyst. The powder obtained is a candidate catalyst for the isomerization of n-butane into isobutene.

### Example 7: Synthesis of an K-Co (Potassium doped Co₃O₄) catalyst

### First procedure:

One precursor bottle with a 18-20% solution of potassium ethoxide or potassium isopropoxide (or a mixture thereof) in isopropanol and one bottle with a solution of cobalt 2-ethylhexanoate in an appropriate solvent is used as precursor reagents for the synthesis of the K-Co based catalysts.

A procedure analogous to the procedure of example 1 is followed to obtain a Potassium doped Co₃O₄ catalyst. The powder obtained is a candidate catalyst for the direct decomposition of N₂O.

### Second procedure:

### One precursor bottle with KOH solution and one bottle with a solution of cobalt 2-ethylhexanoate in an appropriate solvent is used as precursor reagents for the synthesis of the K-Co based catalysts.

A procedure analogous to the procedure of example 1 is followed to obtain a Potassium doped Co₃O₄ catalyst. The powder obtained is a candidate catalyst for the direct decomposition of N₂O.

### Third procedure:

One precursor bottle with 18-20% solution of potassium ethoxide or potassium isopropoxide (or a mixture thereof) in isopropanol and one bottle with a solution of cobalt chloride hexahydrate or cobalt (II) nitrate hexahydrate (or a mixture thereof) in water is used as precursor reagents for the synthesis of the K-Co based catalysts.

A procedure analogous to the procedure of example 1 is followed to obtain a Potassium doped Co₃O₄ catalyst. The powder obtained is a candidate catalyst for the direct decomposition of N₂O.

### Example 8: Synthesis of an K-Ni (Potassium doped Ni) catalyst

### First procedure:

One precursor bottle with a 18-20% solution of potassium ethoxide or potassium isopropoxide (or a mixture thereof) in isopropanol and one bottle with a solution of Nickel(II) hydroacetate or Nickel(II) acetate tetrahydrate (or a mixture thereof) in an appropriate solvent is used as precursor reagents for the synthesis of the K-Ni based catalysts.

### A procedure analogous to the procedure of example 1 is followed to obtain a Potassium doped Ni catalyst. The powder obtained is a candidate catalyst for the selective hydrogenation of 1,6-hexanedinitrile.

### Second procedure:

### One precursor bottle with KOH solution and one bottle with a solution of Nickel(II) hydroacetate or Nickel(II) acetate tetrahydrate (or a mixture thereof) in an appropriate solvent is used as precursor reagents for the synthesis of the K-Ni based catalysts.

A procedure analogous to the procedure of example 1 is followed to obtain a Potassium doped Ni catalyst. The powder obtained is a candidate catalyst for the selective hydrogenation of 1,6-hexanedinitrile.

### Third procedure:

One precursor bottle with a 18-20% solution of potassium ethoxide or potassium isopropoxide (or a mixture thereof) in isopropanol and one bottle with a solution of Nickel(II) nitrate hexahydrate in water is used as precursor reagents for the synthesis of the K-Ni based catalysts.

A procedure analogous to the procedure of example 1 is followed to obtain a Potassium doped Ni catalyst. The powder obtained is a candidate catalyst for the selective hydrogenation of 1,6-hexanedinitrile.

### Example 9: Synthesis of an Li-Mg (lithium doped MgO) catalyst

### First procedure:

One precursor bottle with a solution of Mg acetate tertrahydrate in water and/or methanol and one bottle with a solution of lithium nitrate in water and/or methanol is used as derivatives for the synthesis of the Li-Mg based catalysts.

A procedure analogous to the procedure of example 1 is followed to obtain a lithium doped MgO catalyst. The powder obtained is a candidate catalyst in the oxidative coupling of methane.

### Second procedure:

One precursor bottle with a solution of Mg acetate tertrahydrate in water and/or methanol and one bottle with a solution of lithium ethoxide or lithium isopropoxide in an appropriate solvent is used as derivatives for the synthesis of the Li-Mg based catalysts.

A procedure analogous to the procedure of example 1 is followed to obtain a lithium doped MgO catalyst. The powder obtained is a candidate catalyst in the oxidative coupling of methane.

## Claims

1. An amorphous catalyst or catalyst precursor in the form of a powder having a surface area of from 0.1 m²/g to 50 m²/g which chemical composition comprises oxygen, carbon, hydrogen, optionally nitrogen, and at least a first and a second further elements.

2. The amorphous catalyst or catalyst precursor according to claim 1, wherein said at least a first and a second further elements are independently selected from the list consisting of metalloids, post-transition metals, transition metals, alkaline earth metal, non-metals and alkali metals.

3. The amorphous catalyst or catalyst precursor according to claim 1 or claim 2, wherein said at least a first and a second further elements are a metalloid and a post-transition metal, a metalloid and a transition metal, a non-metal and a transition metal, an alkali metal and a transition metal, an alkaline earth metal and an alkali metal or a post-transition metal and an alkaline earth metal.

4. The amorphous catalyst or catalyst precursor of any one of the preceding claims, wherein said at least a first and a second further elements are Si and Sn, Si and V, Si and Zr, Si and Ti or Al and Mg.

5. The amorphous catalyst of claim 4 wherein said at least two further elements are Si and Sn in a ratio of from 15:1 to 35:1, preferably from 20:1 to 30:1.

6. The amorphous catalyst of claim 4 or claim 5 wherein said at least two further elements are Si and Sn and wherein Sn is predominantly tetracoordinated.

7. A method of producing an amorphous catalyst or catalyst precursor according to any one of claims 1 to 6, said method comprising:
a) Generating an aerosol comprising a mixture of derivatives, said mixture of derivatives comprising at least one derivative per further element, each derivative being a derivative of at least one of said further elements, and at least one of said derivatives being an organic derivative,
b) Optionally diluting said aerosol by a gas, and
c) Passing said aerosol in a plasma generated between two electrodes, at least one of which being provided with a dielectric, thereby forming an amorphous catalyst or catalyst precursor on one of said electrodes.

8. The method according to any one of claims 7 wherein said organic derivatives of said first and second elements are respectively tetra ethyl orthosilicate and titanium tetra isopropoxide, tetra ethyl orthosilicate and tetra-n-butyltin, tetra ethyl orthosilicate and vanadium (V) oxytripropoxide, tetra ethyl orthosilicate and zirconium n-propoxide, or aluminium lactate and magnesium acetate tetrahydrate.

9. The method according to any one of claims 7 or claim 8, wherein said plasma is generated at a power of 0.09 W/cm² or higher, preferably 0.15 W/cm² or higher, more preferably 0.22 W/cm² or higher.

10. The method according to any one of claims 7 to 9, wherein said plasma is generated at a frequency of 11 kHz or lower.

11. An amorphous catalyst or catalyst precursor obtainable by a method comprising:
a) Generating an aerosol comprising a mixture of derivatives, said mixture of derivatives comprising at least one derivative per further element, each derivative being a derivative of at least one of said further elements, and at least one of said derivatives being an organic derivative,
b) Optionally diluting said aerosol by a gas, and
c) Passing said aerosol in a plasma generated between two electrodes, at least one of which being provided with a dielectric, thereby forming said amorphous catalyst or catalyst precursor on one of said electrodes

12. A semi-crystalline catalyst obtainable by calcination of the amorphous catalyst or catalyst precursor of any one of claims 1 to 6 or 11.

13. Use of the amorphous catalyst according to claim 4 to 6 or 11, wherein said at least a first and a second further elements are Si and Sn or Al and Mg, for catalysing the isomerisation of glucose into fructose.

14. Use of the catalyst according to claim 12, wherein said at least a first and a second further elements are Si and V, for catalysing the conversion of methane and carbon dioxide into methanol and formaldehyde.

15. Use of the catalyst according to claim 12, wherein said at least a first and a second further elements are Si and Zr, for catalysing the isomerisation of n-butane into iso-butane.
